(19) **Europäisches Patentamt / European Patent Office / Office européen des brevets**

(11) **EP 1 972 345 B1**

(12) **EUROPEAN PATENT SPECIFICATION**

(45) Date of publication and mention of the grant of the patent:
**13.08.2014 Bulletin 2014/33**

(21) Application number: **05825186.9**

(22) Date of filing: **30.11.2005**

(51) Int Cl.:
**A61K 38/16** [(2006.01)]    **A23L 1/29** [(2006.01)]

(86) International application number:
**PCT/ES2005/000654**

(87) International publication number:
**WO 2007/063141 (07.06.2007 Gazette 2007/23)**

(54) **FOOD PRODUCT FOR ENTERAL OR ORAL NUTRITION**

NAHRUNGSPRODUKT ZUR ENTERALEN ODER ORALEN ERNÄHRUNG

PRODUIT ALIMENTAIRE DESTINE A LA NUTRITION PAR VOIE ORALE OU ENTERALE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**24.09.2008 Bulletin 2008/39**

(73) Proprietor: **Vegenat, S.A.**
**06184 Pueblonuevo del Guadiana, Badajoz (ES)**

(72) Inventors:
- **GIL HERNÁNDEZ, Angel**
  **E-6011 Badajoz (ES)**
- **MARTÍNEZ DE VICTORIA MUÑOZ, Emilio**
  **E-06011 Badajoz (ES)**
- **SAN ROMÁN PAIS, Paloma**
  **E-06011 Badajoz (ES)**
- **RUIZ GUERRERO, Rosa**
  **E-06011 Badajoz (ES)**

(74) Representative: **Gil-Vega, Victor**
**Corazón de Maria, 6**
**28002 Madrid (ES)**

(56) References cited:
**EP-A1- 0 611 568**      **EP-A2- 0 626 175**
**EP-A2- 0 626 176**      **ES-T3- 2 142 842**
**ES-T3- 2 187 595**      **US-A- 5 504 072**
**US-A- 5 514 656**       **US-A- 5 766 621**
**US-A1- 2001 043 958**   **US-A1- 2003 104 033**

- **"Food contg. highly unsatd. fatty acid - comprises omega-3 and omega-6 fatty acids used for treatment of hypertension and cardiac disease", DERWENT, 1989, XP002216491,**
- **DATABASE WPI Week 200601, Derwent Publications Ltd., London, GB; AN 2006-000101, XP003014739 & BR PI0 401 405 A (PIMENTEL GUIMARAES, A.R.) 08 November 2005**

**Description**

This invention relates to a food product for enteral or oral nutrition, comprising a novel protein mixture, a novel lipid mixture, as well as carbohydrates, soluble and insoluble fibre, vitamins and minerals, composing a nutritional product adequate for pathological conditions having some kind of impairment to meet nutritional needs with a normal oral diet.

Enteral nutrition is a type of artificial nutrition that within the context of clinical practice can be defined as that practice that implies providing the body with nutrients by a non usual digestive administration method, always provided that the same is safe and reliable, whether regarding the administration method itself or the nutritive mixture administered, orally (oral-enteral nutrition) or by means of probes (for example nasogastric probe). The two main characteristics of this way of administering nutrients are derived from the suppression of the stages of chewing, salivation and thermal regulation and esophageal digestion as well as the need of special devices. Generally, this type of nutrition is used when food ingestion is impaired and/or temporary or permanent anatomic or functional digestive anomalies are evidenced thus not allowing the adequate transit, digestion or absorption of nutrients.

In the case of oral nutrition, the administration of a manufactured nutrient formula makes up for potential nutritional deficits due to a food intake lower than normal or even in cases where such intake is acceptable, when nutritional needs are highly specific.

Whether with oral or enteral administration products, there is a large number of social groups that require coverage of their nourishing needs with this type of nutritive products to avoid malnutrition. This malnourishment is more frequent in older population, since even when they do not suffer from any specific pathology associated to nutrition, this group presents factors closely related to age, for example decreased number of papillae, lack of teeth that renders mastication difficult, less secretion of saliva and decreased metabolic rate and together with it, a decreased need to eat.

This type of artificial nutrition is also used in several pathological conditions, for example people affected by cerebrovascular accidents, multiple sclerosis, Parkinson's or Alzheimer's diseases, people suffering from mouth or throat cancer or esophageal damage, in general people showing hypermetabolic states with different etiologies (burns, traumas, surgery), chronic illnesses or people who have been subject to prolonged periods of reduced oral intake. All these possible situations have to be taken into account in the case of inpatients, where nutrition control is relatively easier, as well as in the case of outpatients where homecare is in charge of health staff or the patient's relatives or even himself/herself.

All the aforesaid evidences the need for a product for oral or enteral nutrition providing all nutrients necessary to meet nourishing needs and easy to be administered by the customary oral method or by means of probes, with high palatability, especially if it is administered orally.

There are several formulations intended for oral or enteral nutrition, products made of a mixture of nutrients classified within the legal framework as "dietetic products for specific nutritional purposes". These can be complete formulae, supplements and modules. In the case of oral administration formulae, additives such as flavors and aromas are added to improve palatability. Complete formula products, where the nourishing formula contains proteins, carbohydrates, fat, fibre and micro nutrients such as the applicant's T-Diet® range of products are especially important. The prior art disclosed several balanced enteral formulae. US/2003/0104033, for example, describes liquid formulations consisting of stabilized proteins and caseinate, further to other components to enhance preparation creaminess. In these formulae vegetal origin proteins are derived from soy. In the above mentioned document, protein efficiency rates of the protein mixture used are not mentioned. EP 0 626 175 B1 describes liquid nourishing products based on a vegetal protein mixture; however, in this document the need to provide an adequate fat content to grant a complete product is not taken into account. US 5 504 072 describes a complete enteral composition wherein a balanced protein supply and a general fat profile are specified, without contemplating oral administration. Such technical background clarifies the need of a complete product for enteral and oral administration with a high nutritional quality and adapted to meet the needs and requirements of US 2001/0043958 A1 describes a liquid nutritional product useful for providing nutrition to a patient having a malabsorption condition. The nutritional product comprises a protein system including soy protein hydrolysate and partially hydrolyzed caseinate and a structured lipid formed from marine oil and medium chain triglyceride oil. consumers as regards flavour, textures, presentation especially if administered orally.

The nutritional product of this invention, further to having a nutritive composition perfectly specified and homogeneity and fluidity to enable its easy administration by probe and a high palatability enabling an adequate oral administration, has very high indices of protein quality (Protein Efficacy Ratio) that enable higher ease of conversion as compared with highly digestible proteins per excellence, an improved lipid mixture and high digestibility. According to their protein content, products intended for enteral nutrition can be normoproteic (protein proportion is similar to that of a balanced diet, amounting to 12-18% of the total calorie content and with a calories-nitrogen ratio of about non- protein 120-150 kcal /g of nitrogen), hyperproteic (with a protein proportion higher than 18%, with a calories/nitrogen ratio lower than 120) and special (adjusted to specific metabolic conditions). To evaluate the characteristics of the potential enteral diets to be supplied the most important factor to take into consideration is protein content since, as it is well-known, proteins are indispensable for maintaining body mass and cell functionality. Caloric density determines, besides calorie content, the density of the different nutrients comprised in the diet, especially liquid content.

In most of the diets, fats are a concentrated source of energy, they transport fat-soluble vitamins and provide essential fatty acids (at least 3-4%, especially linoleic acid); they can be found as medium-chain triglycerides with variable amounts of long-chain triglycerides. The first ones show a bioavailability as regards digestion, absorption and transportation higher than that of long-chain triglycerides; the second ones provide essential fatty acids. Even though there is competitiveness among them at the time of intestinal absorption, when mixtures of both types are administered the total absorption of the same is increased as compared with the absorption shown when individually administered.

The main types of fibre supplied in enteral nutrition are soluble and insoluble fibre. The first type allows preserving the morphology of intestinal villi, increases lipase activity in the small intestine, increases transit time and, in general, enables normalization of colon functions and improves patient's tolerance. In the second place, insoluble fibre increases faecal mass and reduces constipation.

In all the cases the food product must be adequate for the metabolic needs of the pathologic process in question and must correct pre-existing deficits. Likewise, it must meet palatability requirements, being this a determinant condition for oral administration; monotony must be avoided as well as undesired environmental stimuli, for example unpleasant odours of some of the components, etc.

The novel food product for oral or enteral nutrition object of this invention comprises a new protein mixture based on caseinate, pea protein and milk serum proteins enriched with glycomacropeptide, a lipid mixture comprising several stabilized vegetal and purified fish oils, as well as carbohydrates, soluble and insoluble fibres, vitamins and minerals. This is a dietary product for nutritional use adequate for pathologic conditions wherein some type of impairment to satisfy nutritional needs by means of a normal oral diet is present. The protein mixture contained in the novel food product for oral or enteral nutrition contains caseinate (50%), pea protein (25%) and milk serum protein (25%), the latter enriched with glycomacropeptide.

This new protein mixture based on caseinate, pea protein and milk serum proteins enriched with glycomacropeptide enables a singular amino acid composition, very similar to that of proteins considered nutritionally as standard reference proteins (egg proteins). Amino acid profile is shown below in Table 1:

**Table 1**

| Amino acid profile | per 100 g of protein |
|---|---|
|  |  |
| Alanine ALA | 4.02 |
| Arginine ARG | 4.92 |
| Aspartic acid ASP | 9.20 |
| Cystine CYS | 1.05 |
| Glutamic acid GLU | 22.68 |
| Glycine GLY | 2.50 |
| Histidine HIS | 2.52 |
| Isoleucine ILE | 5.52 |
| Leucine LEU | 9.67 |
| Lysine LYS | 8.32 |
| Methionine MET | 2.47 |
| Phenylalanine PHE | 4.87 |
| Proline PRO | 9.09 |
| Serine SER | 5.27 |
| Threonine THR | 4.85 |
| Tryptophane TRP | 1.32 |
| Tyrosine TYR | 4.57 |
| Valine VAL | 6.10 |
| MET+Cystein | 3.52 |

(continued)

| Amino acid profile | per 100 g of protein |
|---|---|
| PHE+Tyrosine | 9.44 |

The protein mixture of the food product of the invention shows that its growth coefficient efficiency and other biologic quality parameters are much higher than those of the standard protein used as reference in biological assays as evidenced by the exemplary embodiment described below. This novel protein mixture provides amino acid nitrogen in enough quantity and quality and likewise, since glycomacropeptide is incorporated as well as sialic acid (N-acetylneuraminic acid), the administration of the food product of the invention has the advantage of its prebiotic nature, modulating intestinal macrobiota by means of increasing the number of bifidobacteria. Bifidobacteria proliferation has numberless effects, among them decreasing intestinal pH reducing or even inhibiting the growth of harmful bacteria, activating peristaltic movements and the immune system.

The food product according to this invention contains a lipid mixture as well. Such lipid mixture consists of a mixture of several vegetal oils (98.4%) and purified fish oils (1.6%). This mixture facilitates a fatty acid profile with a relatively low content of saturated fatty acids, a high content of medium-chain and monounsaturated fatty acids (oleic acid) and a balanced supply of essential (linoleic and $\alpha$-linolenic) fatty acids, as well as of long-chain polyunsaturated fatty acids such as those of the series Omega 3 (n-3). This mixture has been used to develop optimal plasma and cell fatty acid profiles that limit the formation of oxidative compounds in blood plasma and at cell level as well as the formation of certain biological factors involved in metabolic syndrome. The lipid profile of the food product according to this invention is show in Table 2 below:

Table 2

| Lipid profile | g per 100 g of fat |
|---|---|
| C 8:0 (caprylic acid) | 4.92 |
| C 10:0 (capryc acid) | 4.43 |
| C 12:0 (lauric acid) | 0.25 |
| C 14:0 (myristic acid) | 0.36 |
| C 15:0 (pentadecanoic acid) | 0.025 |
| C 16:0 (palmitic acid) | 12.07 |
| C 16:1 (palmitoleic acid) | 0.14 |
| C 17:0 (heptadecanoic acid) | 0.025 |
| C 18:0 (estearic acid) | 3.00 |
| C 18:1 (oleic acid) | 49.59 |
| C 18:2 (linoleic acid) | 19.95 |
| C 18:3 (linolenic acid) | 2.28 |
| C 20:0 (arachidic acid) | 0.43 |
| C 20:1 (c-11-eicosenoic acid) | 1.51 |
| EPA | 0.32 |
| C 22:0 (behenic acid) | 0.34 |
| DHA | 0.16 |
| C 24:0 (lignoceric acid) | 0.15 |

Medium-chain fatty acids (MCFA), Target: 10.3% Range (min-max): 8.5-10.3%. The relatively high amount of these MCFA in the formula provides a significant part of the total energy of the diet, easily absorbable without the aid of pancreatic lipase, while facilitating obtaining tissue energy since these fatty acids are oxidized in the mitochondrion without passage facilitated by the carnitine -dependant transport system.

The fatty acid profile present in the food product of this invention provides a monounsaturated:saturated fatty acid ratio of 2:1, being the presence of oleic acid (monounsaturated) majority, and being saturated fatty acids mainly constituted by medium-chain fatty acids with very low values of lauric and myristic acids.

    Lauric and myristic fatty acids: Target: 0.61 %; Maximum range 1.0%
    Proportion / MCFA: Range (min- max): 0-0.12%

Lauric and myristic acids are strongly atherogenic and the quantity of the same is limited to enhance the healthy cardiovascular properties of the oil mixture present in the food product of the invention.
Likewise the proportion of stearic acid is quite lower than in other formulations of this type of preparations. This lipid mixture present in the product of the invention has a high bioavailability thanks to its relatively high content of short-chain fatty acids and it is not very atherogenic; in this regard there are several scientific evidences indicating that a high content of oleic acid decreases LDL cholesterol and increases HDL cholesterol, being the presence of the most atherogenic acids known (C 12:0 and C 14:0) limited.

    Saturated fatty acids >14 C : Target: 15.8%; Range (min- max): 12-21%
    Total saturated fatty acids: Target: 26.71%; Range (min- max): 20-32%
    Palmitic acid: Target: 12%; Range (min- max): 10-15%
    Stearic acid: Target: 3%; Maximum 5%
    Arachidic acid: Target: 0.43%; Maximum 0.5%
    Behenic acid: Target: 0.34%; Maximum 0.5%

The limit in the total amount of saturated fatty acids above 14C and in the total fatty acids is due to the importance of limiting the atherogenicity of the lipid mixture.

    Monounsaturated fatty acids (Oleic acid): Target 49.59%; Range (min-max): 45-55%
    Ratio total MUFA/SFA Target: 1.84% Range (min-max): 1.6-2.0

The high content of oleic acid in the formula is aimed at boosting the healthy cardiovascular effects of the lipid mixture (they increase HDL cholesterol), as well as at favouring its antioxidant effect.
Linoleic acid contribution is about 7% of the energy and $\alpha$-linolenic acid contribution is 0.7%, being the ratio between these two fatty acids of 1/10. This meets the most modern criteria (report by the WHO on fatty acids needs) of supplementing fatty acids to cover the requirements of essentials fatty acids. Many mixtures currently in use for enteral or oral nutrition do not have adequate amounts of $\alpha$-linolenic acids; in other formulations the amounts of linoleic acid are excessive and in many of them the ratio between these two acids is inadequate.
In the lipid mixture of the product according to the invention, MUFA:polyunsaturated fatty acids ratio (PUFA) is about 2. This prevents an excessive unsaturation of cell membranes and limits their oxidation. On the other hand, it is well-known that an excessive intake of linoleic acid inhibits the formation of long-chain PUFA at intestinal and hepatic levels.
Total poly-unsaturated fatty acids (PUFA): Target 22.7%, Range (min-max): 22-25%

    Linoleic acid          Target: 19.95%          Range (min- max): 17.1- 22.8%
    $\alpha$-Linolenic acid    Target: 2.28%          Range (min- max): 1.7- 2.4%
    Eicosapentaenoic acid (EPA):    Target 0.32%;          Range (min-max): 0.3-0.34%
    Docosahexaenoic acid (DHA):    Target 0.16%;          Range (min-max): 0.3-0.4%
    Proportion MUFA/PUFA          Target: 2.18          Range (min- max): 1.8-2.4
    Proportion PUFA/SFA          Target: 0.85          Range (min- max): 0.75-1.00
    Proportion EPA/DHA          Target: 2          Range (min- max): 1.8-2.2

The lipid mixture present in the food product of the invention has been supplemented with adequate quantities of eicosapentaenoic acid (EPA) and docosahexaenoic acid (DHA) in a 2/1 ratio. The contribution of these long-chain PUFA of the n-3 series (Omega 3) limits the formation of triglycerides, which levels are high in the metabolic syndrome, exert a role as potent antiinflammatory agents and contribute to limit platelet aggregation. In this sense, its use in certain populations such as older people is very interesting since they usually suffer cardiovascular conditions and other chronic inflammatory pathologies.
The mixture of oils is conveniently stabilized using a mixture of soy lecithin and $\alpha$-tocopherol to limit the oxidation of its components and to limit the biologic oxidation experienced by consumers of this food product.

The food product of the invention contains carbohydrates as well. These are presented as a mixture of maltodextrin together with a soluble and insoluble fibre mixture made of soluble fructooligosaccharides and cellulose derivatives. Such fibre combination allows to preserve the morphology of intestinal villi, increases lipase activity in the small intestine, increases transit time and in general, enables normalization of colon functions and improves patient's tolerance. Cellulose derivatives increase faecal mass and improve constipation. Likewise it contains minerals and vitamins, and the amounts thereof meet the recommendations made by the American Institute of Medicine (IOM 1998-2005).

The following table shows a summary of component quantity as well as the average nutritional value of the food product of the invention pursuant to an embodiment of the same (hyperproteic product).

|  | Per 100 mL |  | Per 100 mL |
|---|---|---|---|
| **Average nutritional information** |  |  |  |
| **Energetic value** | 125 kcal |  |  |
|  | 522 kjul |  |  |
| **Proteins** | 6.3 g | **Vitamins** |  |
| **Carbohydrates** | 13.5 g | A | 60.00 μg |
| from which, sugar | 1.0 g | D | 1.00 μg |
| **Fats** | 4.19 g | E | 1.00 mg |
| From which |  | C | 6.00 mg |
| Saturated fat, from which | 1.27 g | B1 | 0.08 mg |
| MCT | 0.46 g | B2 | 0.10 mg |
| monounsaturated | 2.52 g | B3 | 1.13 mg |
| Polyunsaturated, from which | 1.11 g | B6 | 0.11 mg |
| Linoleic acid | 0.98 g | B9 | 26.67 μg |
| Linolenic acid | 0.11 g | B12 | 0.16 μg |
| EPA | 0.013 g | Biotin | 1.33 μg |
| DHA | 0.006 g | Pantothenic acid | 0.97 mg |
|  |  | K | 8.00 μg |
| **Dietary fibre** | 2.0 g |  |  |
| **Minerals** |  |  |  |
| Calcium | 80.00 mg | Manganese | 0.15 mg |
| Phosphorus | 46.67 mg | Fluoride | 0.19 mg |
| Potassium | 250.00 mg | Molybdenum | 4.40 mg |
| Sodium | 83.33 mg | Chromium | 2.00 μg |
| Chloride | 126.67 mg | Selenium | 3.67 μg |
| Iron | 0.83 mg | Magnesium | 24.67 mg |
| Zinc | 0.73 mg | Iodine | 10.00 μg |
| Copper | 76.67 μg |  |  |

The following table shows a summary of components quantity as well as the average nutritional value of the food product of the invention pursuant to another embodiment of the same (normoproteic product).

|  |  | Per 100 mL |  |
|---|---|---|---|
| **Average nutritional information** |  |  |  |
| **Energetic value** | kcal | 100 |  |

(continued)

|  | | Per 100 mL | | | |
|---|---|---|---|---|---|
| **Average nutritional information** | | | | | |
|  | kJ | 420 | | | |
| **Proteins** | g | 4.0 | **Vitamins** | | Per 100 mL |
| **Carbohydrates** | g | 12.3 | A | µg | 60,00 |
| from which, sugar | g | 0.8 | D | µg | 1.00 |
| **Fats** | g | 3.9 | E | mg | 1.00 |
| From which | | | C | mg | 6.00 |
| Saturated fat, from which | g | 0.93 | B1 | mg | 0.08 |
| MCT | g | 0.39 | B2 | mg | 0.09 |
| Monounsaturated | g | 2.02 | B3 | mg | 1.07 |
| Polyunsaturated, from which | g | 0.95 | B6 | mg | 0.12 |
| Linoleic acid | g | 0.78 | B9 | µg | 26.67 |
| Linolenic acid | g | 0.08 | B12 | µg | 0.16 |
| EPA/DHA | g | 0.01 | Biotin | µg | 1.34 |
| **Dietary fibre** | g | 1.7 | Pantothenic acid | mg | 0.80 |
| **Minerals** | | | K | µg | 8.00 |
| Calcium | mg | 70.00 | | | |
| Phosphorus | mg | 65.00 | | | |
| Potassium | mg | 250.00 | | | |
| Sodium | mg | 83.33 | | | |
| Chloride | mg | 126.67 | | | |
| Iron | mg | 0.83 | | | |
| Zinc | mg | 0.73 | | | |
| Copper | µg | 60.00 | | | |
| Iodine | µg | 10.00 | | | |
| Selenium | µg | 3.67 | | | |
| Magnesium | mg | 24.67 | | | |
| Manganese | mg | 0.15 | | | |
| Fluoride | mg | 0.19 | | | |
| Molybdenum | µg | 3.67 | | | |
| Chromium | µg | 2.00 | | | |

The food product of the invention contains also the usual additives used in this type of food preparations depending on its presentation form, preferably liquid. In order to meet the high palatability requested by consumers, the food product of the invention may contain, further to the stabilizers already mentioned, flavours, aromas and seasonings. In one embodiment of the product, it contains colours and vanilla flavour.

In one embodiment according to the invention, the product is presented as a normoproteic diet in liquid presentation with two caloric densities, 1 kcal/mL and 1.5 kcal/mL. In another embodiment, the food product of the invention is presented as a hyperproteic diet in liquid presentation with two caloric densities, 1 kcal/mL and 1.25 kcal/mL.

The invention product is described in further detail through the following exemplary embodiment.

Evaluation of the nutritive quality of the protein mixture contained in the productof the invention and the methods to prepare its normoproteic and hyperproteic embodiments

The protein used in this exemplary embodiment is a combination of animal protein (75%) and vegetal protein (25%), more specifically 50% of caseinate, 25% of milk serum proteins and 25% of pea protein.

Method

*Experimental design*

The study was carried out in two phases, the first one in the Physiology Laboratory of the Pharmacy School of the University of Granada and the second one in the Animal Facilities of the same institution. In the first phase the protein mixture to be used in the enteral nutrition product of the invention was analyzed and in the second phase a product already produced at two different protein concentrations (normoproteic and hyperproteic), freeze-dried and adapted to meet rat nutrient requirements.
Each experiment lasted 10 days, 3 days for adapting the animals to the diet and environmental conditions and 7 days to quantify solid intake, changes in weight and faeces and urine collection.

*Materials and Methods*

*Animals:*

Twenty 21-day-old *Wistar* rats, 10 males and 10 females, were used in each experiment. The rats supplied by the Granada University Animal Facilities, with an average weight of between 50 g and 52 g were allocated into two groups having 5 females and 5 males each, .
The rats were placed in individual metabolic cages in a room at 23°C and with a light-darkness photoperiod of 12 hours. The diet and water were supplied daily *ad libitum.*
The diet was prepared at the Granada University Animal facilities pursuant to the recommendations of the American Institute of Nutrition (AIN), based on AIN-93G diet (Reeves, PG 1997) and making any necessary change when any nutrient was modified to adjust it to the rats' requirements.

Control and experimental diet composition in the first experiment

| Components | Diets (%) | |
| --- | --- | --- |
| | Control | Experimental |
| Protein | 12 (casein) | 12* |
| Corn starch | 47.75 | 47.75 |
| Dextrinated starch | 13.2 | 13.2 |
| Sucrose | 10 | 10 |
| Soy oil | 7 | 7 |
| Cellulose (fibre) | 5 | 5 |
| AIN-93G-MX mineral supplement | 3.5 | 3.5 |
| AIN-93-VX vitamin supplement | 1 | 1 |
| L-cystine | 0.18 | 0.18 |
| Choline bitartrate | 0.25 | 0.25 |
| Antioxidant (mg) | 0.0014 | 0.0014 |
| *Assayed protein: 50% Potassium caseinate, 25% milk serum proteins and 25% pea protein. | | |

Components of diets adapted to meet the rats' requirements with freeze-dried normoproteic and hyperproteic enteral nutrition products

| Components | Diets (%) | |
|---|---|---|
| | Normoproteic | Hyperproteic |
| Freeze-dried product | 68.97 | 56.07 |
| Corn starch | 11.10 | 24 |
| Sucrose | 10 | 10 |
| Cellulose (fibre) | 5 | 5 |
| AIN-93G-MX mineral supplement | 3.5 | 3.5 |
| AIN-93-VX vitamin supplement | 1 | 1 |
| L-cystine | 0.18 | 0.18 |
| Choline bitartrate | 0.25 | 0.25 |
| Antioxidant (mg) | 0.0014 | 0.0014 |

*Sample treatment:*

Collected faeces were frozen at -20°C, and then they were freeze-dried and ground. Urine was collected in a 5% HCl solution with Xiro-Taxiro indicator to avoid nitrogen losses.

*Analysis techniques:*

Nitrogen determination was carried out through the Kjeldahl method, using 0.5 g of dried, ground faeces, 5 mL of urine and 0.5 g of diet.

*Calculated Indices:*

Protein Efficiency Ratio (PER): This index is used to determine protein quality in accordance with animal weight gain.

$$PER = \frac{Weight\ gain}{Protein\ intake}$$

Apparent Digestibility Coefficient (ADC): Digestibility is a measure of food nutritive value, its ease of conversion into useful nutrients by the digestive tract. Apparent digestibility does not include excretion of endogenous origin faeces.

$$CDA = \frac{NI - NHe}{NI} \times 100$$

Percentage of Retention R/A: Retention indicates the metabolic use of amino acids and it is apparent whenever the nutrient fraction of endogenous origin is not taken into account. It is the ratio between retained and absorbed protein.

$$RP = \frac{Retained}{Absorbed} \times 100$$

$$RP = \frac{(NI - NHe) - (NOr)}{(NI - NHe)} \times 100$$

Retained Intake Ratio: Evidences protein intake incorporated into the human body, it is the retained protein intake that is used.

$$RI = \frac{Retained}{Ingested} \times 100$$

$$RI = \frac{(NI - NHe) - (NOr)}{NI} \times 100$$

Results

*Growth, weight gain and food intake:*

Animal weight at the beginning and at the end of the first experiment was 58.52 g and 108.53 g for the experimental protein group and 56.30 g and 108.37 g for the control group respectively. In the second experiment, normoproteic and hyperproteic groups evidenced weight gains as from 42.30 g and 41.30 g, to 93.5 g and 92.40 g, respectively.

*Studied Indices:*

The following table shows a summary of the indices calculated to measure the quality of the protein mixture under study.

Protein Efficiency Ratio (PER), Apparent Digestibility Coefficient (ADC), Percentage of Retention (R/A) and Retained/Ingested Ratio (R/I) of the four groups under study

| Group | PER | ADC (%) | % RIA: | % R/I |
|---|---|---|---|---|
| Control Experimental | $3.88 \pm 0.61$ | $93.8 \pm 1.15$ | $84.00 \pm 3.93$ | $78.82 \pm 4.41$ |
| Protein | $4.04 \pm 0.29$ | $93.91 \pm 0.69$ | $85.33 \pm 4.51$ | $80.14 \pm 4.53$ |
| Normoproteic | $3.35 \pm 0.53$ | $90.63 \pm 0.77$ | $76.13 \pm 2.54$ | $68.99 \pm 2.03$ |
| Hyperproteic | $2.85 \pm 0.42$ | $91.22 \pm 0.68$ | $75.21 \pm 4.94$ | $68.63 \pm 4.82$ |

Note: average $\pm$ standard deviation.

It is to be noted that all PER valves are above 2.5, and from higher to lower ranking we find the experimental group, the control group, the normoproteic group and the hyperproteic group. The European Society of Pediatric Gastroenterology, Hepatology and Nutrition established a PER value of 2.55 as the minimum nutritional requirement for infant milk formulas and by way of similarity for enteral nutrition diets. As it can be clearly seen, apparent digestibility coefficient of the protein mixture of the invention (the experimental group) has the highest value, 93.91%, very similar to that of the control group, 93.8%. Hyperproteic and normoproteic group values are 91.22% and 90.63% respectively. In the case of the Percentage of Retention and the Retained/Ingested Protein Ratio, their behaviour is very similar. Experimental and control groups are very similar and their values are higher than hyperproteic and normoproteic groups, which in turn show very similar values between them.

Discussion

*Growth, weight gain and food intake:*

Animal growth was increased in the four groups under study during the experiment, however, growth rate decreased during the last two days, without affecting their normal development. The Group with the highest weight gain was the experimental group, thus indicating that the diet used is adequate as compared with that of the control Group and its quality is good to maintain animal growth rate.

Food intake was clearly reflected in growth.

*Analyzed Indices:*

The experimental protein mixture had the highest PER calculated by us. In similar studies, Proll J. et al. in 1998 and Ghulan S. in 1997 reported a high PER value for casein, but lower than that obtained by us for the protein mixture of the invention. Van Dael P et al. (2005) and Silva et al. (2003) had a PER value for casein lower than that reported by the above mentioned authors and lower than that found by us for the group with the lowest PER value (hyperproteic group).

It is worth noting that nitrogen consumption values as compared between the control group and the experimental group had little variations; however, PER values for the experimental group were much higher, thus evidencing the better quality of the protein mixture.

Millward DJ and Jackson AA (2003) found that digestibility of egg, milk, meat and soy isolated protein is higher than 95%, and digestibility of cereals, peas and rice is between 80% and 90%. The values found by us are 93.8% for the control diet (casein) and 93.91% for the protein mixture of the invention (experimental protein). Proll J et al. (1998) reported an apparent digestibility for casein of 91.2% (lower than the values reported by us for the experimental protein) and a true digestibility of 98.5%. Van Dael P et al. (2005) and Silva et al. reported a true digestibility for casein of 97% and 99.19% respectively. Having found apparent digestibility values of about 94% and knowing that upon making corrections for true digestibility those values are increased, we can state that the ease of conversion of the protein under study is very good as compared to highly digestible proteins per excellence.

Normoproteic and hyperproteic diets had digestibility values lower than those of the groups mentioned above, probably due to the losses suffered on account of thermal treatment, but they were always above 90%

Percentage of retention (RP) and retained/ingested ratio (R/I) values are apparent values since endogenous protein values are not considered. However, some findings regarding Biologic Values (BV) and Net Protein Use (NPU) values are equivalent to those but they are true values and not apparent. RP and R/I values reported by us for the experimental protein are higher than those reported by Van Dael P et al. in 2005, for casein Biologic Value and NPU. In a publication issued by the FAO (1981) including several assays, an average BV of 79.7% and an average NPU of 72.1% for casein were reported; those values were lower than those found by us for the experimental protein and slightly higher than those of normo- and hyperproteic diets. Therefore we can assume that the efficacy of the experimental protein ingested and used by the body is very high and adequate for growth and development.

It is worth noting that control and experimental diets are diets specially designed for rats containing all components and a palatability accepted by these animals. Normoproteic and hyperproteic diets are intended for human use and have been adapted to cover the nutritional requirements of these animals. However, they have undergone technical processes (mixing, thermal treatment and freeze-drying) that modify food organoleptic characteristics above all, and thus performance of indices calculated may not have been as good as that of the experimental group in spite of having the same protein mixture Therefore, we may conclude that the quality of the protein mixture included in the food products of the invention is excellent and adequate for enteral or oral nutrition.

**Claims**

1. A food product intended for enteral or oral nutrition containing a protein mixture, a lipid mixture as well as carbohydrates, soluble and insoluble fibre, vitamins and minerals, **characterized in that**

  a) The protein mixture is used in an amount between 4 and 6,3 g/100 mL of food product and contains caseinate 50%, pea proteins 25% and milk serum proteins 25%, with the following amino acid profile: Ala 4.02; Arg 4.92; Asp 9.20; Cys 1.05; Glu 22.68; Gly 2.50; His 2.52; Ile 5.52; Leu 9.67; Lis 8.32; Met 2.47; Phe 4.87; Pro 9.09; Ser 5.27; Thr 4.85; Trp 1.32; Tyr 4.57; Val 6.10; Met+cystein 3.52; Phe+tyrosine 9.44.
  b) The lipid mixture contains vegetable oils and a fish oil, having such mixture the following fatty acid proportion: Medium-chain fatty acids (MCFA): 10.3%, [(min-max): 8.5-10.3%]; Lauric and myristic fatty acids: 0.61% [maximum 1.0%]; proportion/MCFA: 0-0.12%; saturated fatty acids >14C: 15.8%, [(min-max): 12-21%]; total saturated fatty acids: 26.71%, [(min-max): 20-32%]; monounsaturated fatty acids: 49.59%, [(min-max): 45-55%]; proportion MUFA/Total SFA: 1.84%, [(min-max): 1.6-2.0]; Total poly-unsaturated fatty acids (PUFA): 22.7%, [(min-max): 22-25%]; proportion MUFA/PUFA: 2.18 [(min-max): 1.8-2.4%]; proportion PUFA/SFA: 0.85 [(min-max): 0.75-1.00]; proportion EPA/DHA:2 [(min-max): 1.8-2-2];

c) The ratio proteins to lipids is between 1 and 1,5.

**2.** A food product according to claim 2, **characterized in that** milk serum proteins contained in the protein mixture are enriched with glycomacropeptide.

**3.** A food product according to claim 2 **characterized in that** the protein mixture has a protein efficacy index of about 4.04 $\pm$ 0.29.

**4.** A food product according to claim 1, **characterized in that** the lipid mixture has a fatty acid profile with low content of saturated fatty acids, high content of medium-chain and monounsaturated fatty acids and a balanced supply of essential (linoleic and -linolenic) fatty acids, as well as of long-chain polyunsaturated fatty acids from the Omega 3 (n-3) series and 98.4% comes from vegetable oils and 1.6% from fish oil.

**5.** A food product according to claim 1, **characterized in that** is in liquid form.

**6.** A food product according to the above claims **characterized in that** it is a normoproteic diet with a caloric density of 1 kcal/mL.

**7.** A food product according to the above claims **characterized in that** it is a normoproteic diet with a caloric density of 1.5 kcal/mL.

**8.** A food product according to claims 1-6 **characterized in that** it is a hyperproteic diet with a caloric density of 1 kcal/mL.

**9.** A food product according to claims 1-6 **characterized in that** it is a hyperproteic diet with a caloric density of 1.25 kcal/mL.

**10.** A food product according to any of the above claims **characterized in that** it includes flavours and colours to grant high palatability.

**Patentansprüche**

**1.** Ein Lebensmittelprodukt für enterale oder orale Ernährung, das eine Proteinmischung, eine Lipidmischung sowie Kohlenhydrate, lösliche und unlösliche Ballaststoffe, Vitamine und Mineralien enthält, **gekennzeichnet dadurch, dass**

a) die Proteinmischung in einer Menge von 4 bis 6,3 g/100 mL des Lebensmittelprodukts verwendet wird und 50% Kaseinat, 25% Kichererbsenproteine und 25% Proteine des Milchserums enthält mit nachstehendem Aminosäurenprofil: Ala 4,02; Arg 4,92; Asp 9,20; Cys 1,05; Glu 22,68; Gly 2,50; His 2,52; Ile 5,52; Leu 9,67, Lis 8,32; Met 2,47; Phe 4,87; Pro 9,09; Ser 5,27; Thr 4,85; Trp 1,32; Tyr 4,57; Val 6,10; Met+Cystein 3,52, Phe+Thyrosin 9,44.
b) die Lipidmischung Planzen- und Fischöle enthält, wobei diese Mischung folgende Fettsäurenproportion aufweist: mittelkettige Fettsäuren (MCFA): 10,3%, [(min-max): 8,5-10,3%]; Laurin- und Myristinfettsäuren: 0,61% [Maximal 1,0%); Proportion/MCFA: 0-0,12%; gesättigte Fettsäuren >14C: 15,8%, [(min.-max.): 12-21 %]; insgesamt gesättigte Fettsäuren: 26,71 %, [(min.-max.): 20-32 %]; einfach ungesättigte Fettsäuren: 49,59 %, [(min.-max.): 45-55 %]; Proportion MUFA/SFA Total: 1,84 % [(min.-max.): 1,6-2,0]; insgesamt mehrfach ungesättigte Fettsäuren (PUFA): 22,7 %, [(min.-max.): 22-25 %]; Proportion MUFA/PUFA: 2,18 [(min.-max..): 1,8-2,4 %]; Proportion PUFA/SFA: 0,85 [(min.-max.): 0,75-1,00]; Proportion EPA/DHA: 2 [(min.-max.): 1,8-2-2];
c) das Verhältnis von Proteinen zu Lipidenzwischen 1 und 1,5 liegt.

**2.** Ein Lebensmittelprodukt gemäss Anspruch 2, **gekennzeichnet dadurch, dass** die Milchserumproteine in der Proteinmischung mit Glycomakropeptid angereichert sind.

**3.** Ein Lebensmittelprodukt gemäss Anspruch 2, **gekennzeichnet dadurch, dass** die Proteinmischung einen Wirksamkeitsindex der Proteine von ca. 4,04 ± 0,29 aufweist.

**4.** Ein Lebensmittelprodukt gemäss Anspruch 1, **gekennzeichnet dadurch, dass** die Lipidmischung ein Fettsäurenprofil hat mit einem geringen Anteil an gesättigter Fettsäure, einem hohen Gehalt an mittelkettigen und einfach ungesättigten Fettsäuren und einer ausgewogenen Versorgung der wesentlichen (Linol- und Linolen-) Fettsäuren, sowie mit langkettigen mehrfach ungesättigten Fettsäuren der Omega 3 (n-3) - Gruppe und 98,4 % Öle pflanzlichen Ursprungs sowie 1,6 % Fischöl.

**5.** Ein Lebensmittelprodukt gemäss Anspruch 1, **gekennzeichnet dadurch, dass** es in flüssiger Form vorliegt.

**6.** Ein Lebensmittelprodukt gemäss vorhergehenden Ansprüchen, **gekennzeichnet dadurch, dass** es einer Normoproteindiät mit einer Energiedichte von 1 kcl/mL entspricht.

**7.** Ein Lebensmittelprodukt gemäss vorhergehenden Ansprüchen, **gekennzeichnet dadurch, dass** es einer Normoproteindiät mit einer Energiedichte von 1,5 kcl/mL entspricht.

**8.** Ein Lebensmittelprodukt gemäss einem der Ansprüche 1-6, **gekennzeichnet dadurch, dass** es einer Hyperproteindiät mit einer Energiedichte von 1 kcal/mL entspricht.

**9.** Ein Lebensmittelprodukt gemäss einem der Ansprüche 1-6, **gekennzeichnet dadurch, dass** es einer Hyperproteindiät mit einer Energiedichte von 1,25 kcal/mL entspricht.

**10.** Ein Lebensmittelprodukt gemäss einem der obigen Ansprüche gekennzeichnet dardurch, dass es Arom- und Farbstoffe enthält, um eine grosse Schmackhaftigkeit zu gewähren.

## Revendications

**1.** Un produit alimentaire destiné à la nutrition orale ou entérale, contenant un mélange de protéines, un mélange de lipides, ainsi que des hydrates de carbone, des fibres solubles et insolubles, des vitamines et des minéraux, **caractérisé en ce que**

a) Le mélange de protéines est utilisé dans une proportion de 4 à 6,3 g/100 mL de produit alimentaire et contient 50 % de caséinate, 25 % de protéines de pois et 25 % de protéines de lactosérum, avec le profil en acides aminés suivant : Ala 4,02 ; Arg 4,92 ; Asp 9,20 ; Cys 1,05 ; Glu 22,68 ; Gly 2,50 ; His 2,52 ; Ile 5,52 ; Leu 9,67 ; Lis 8,32 ; Met 2,47 ; Phe 4,87 ; Pro 9,09 ; Ser 5,27 ; Thr 4,85 ; Trp 1,32 ; Tyr 4,57 ; Val 6,10 ; Met+cystéine 3,52 ; Phe+tyrosine 9,44.
b) Le mélange de lipides contient des huiles végétales et une huile de poisson, ce mélange ayant la proportion d'acides gras suivante : acides gras à chaîne moyenne (AGCM) : 10,3 % [(min-max) : 8,5-10,3 %] ; acides gras myristiques et lauriques : 0,61 % [maximum 1,0 %] ; proportion/AGCM : 0-0,12 % ; acides gras saturés > 14C : 15,8 % [(min-max) : 12-21 %] ; total acides gras saturés : 26,71 % [(min-max) : 20-32 %] ; acides gras monoinsaturés : 49,59 %, [(min-max): 45-55 %] ; proportion AGMI/total AGS: 1,84 %, [(min-max) : 1,6-2,0] ; total acides gras polyinsaturés (AGPI) : 22,7 %, [(min-max) : 22-25 %); proportion AGMI/AGPI: 2,18 [(min-max) : 1,8-2,4 %] ; proportion AGPI/AGS : 0,85 [(min-max) : 0,75-1,00] ; proportion EPA/DHA : 2 [(min-max) : 1,8-2,2] ;
c) Le rapport des protéines aux lipides oscille entre 1 et 1,5.

**2.** Un produit alimentaire selon la revendication 2 **caractérisé en ce que** les protéines du lactosérum que renferme le mélange de protéines sont enrichies de glycomacropeptide.

**3.** Un produit alimentaire selon la revendication 2 **caractérisé en ce que** le mélange de protéines possède un coefficient d'efficacité protéique d'environ 4,04 ± 0,29.

**4.** Un produit alimentaire selon la revendication 1 **caractérisé en ce que** le mélange de lipides a un profil d'acides gras avec une teneur faible en acides gras saturés, une teneur élevée en acides gras monoinsaturés et à chaîne moyenne et un apport équilibré en acides gras indispensables (linoléique et linolénique), ainsi que des acides gras polyinsaturés à longue chaîne provenant de séries oméga-3 (n-3), dont 98,4 % provient d'huiles végétales et 1,6 % d'huile de poisson.

**5.** Un produit alimentaire selon la revendication 1 **caractérisé en ce que** sa présentation est liquide.

**6.** Un produit alimentaire selon les revendications susmentionnées **caractérisé en ce qu'**il s'agit d'un régime normoprotéique avec une densité calorique de 1 kcal/mL.

**7.** Un produit alimentaire selon les revendications susmentionnées **caractérisé en ce qu'**il s'agit d'un régime normoprotéique avec une densité calorique de 1,5 kcal/mL.

**8.** Un produit alimentaire selon les revendications 1 à 6 **caractérisé en ce qu'**il s'agit d'un régime hyperprotéique avec une densité calorique de 1 kcal/mL.

**9.** Un produit alimentaire selon les revendications 1 à 6 **caractérisé en ce qu'**il s'agit d'un régime hyperprotéique avec une densité calorique de 1,25 kcal/mL.

**10.** Un produit alimentaire selon chacune des revendications susmentionnées **caractérisé en ce qu'**il inclut des saveurs et des couleurs qui garantissent une appétence élevée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030104033 A
- EP 0626175 B1
- US 5504072 A
- US 20010043958 A1

**Non-patent literature cited in the description**

- IOM. American Institute of Medicine, 1998